# EUROPEAN PATENT APPLICATION

(11) **EP 4 768 016 A1**
(43) Date of publication of application: **01.07.2026**
(21) Application number: 25209997.3
(22) Date of filing: 21.10.2025
(51) Int. Cl.: A61K 9/00, A61K 9/20

(54) **SUSTAINED-RELEASE FAMPRIDINE COMPOSITION WITH HIGH FLOWABILITY AND MOISTURE CONTROL**

(30) Priority: 25.12.2024 TR 2024020491
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); IPEK, Celaleddin, Istanbul (TR); PEHLIVAN AKALIN, Nur, Istanbul (TR); ULUSOY BOZYEL, Muge, Istanbul (TR); YENI, Songul, Istanbul (TR); DAGCIOGLU, Erdal, Istanbul (TR)
(74) Representative: Genç Ilhan, Oznur

(57) **Abstract**

The present invention relates to a sustained-release fampridine composition with high flowability and moisture control which can be used to treat individuals affected with multiple sclerosis and a preparation process thereof.

## Description

### Field of the Invention

The present invention relates to a sustained-release fampridine composition with high flowability and moisture control which can be used to treat individuals affected with multiple sclerosis and a preparation process thereof.

### Background of the Invention

Multiple sclerosis (MS) is a progressive neurological disease that damages the central nervous system by targeting the myelin sheath. The demyelination of nerve fibers disrupts electrical signals, causing delayed or blocked nerve transmission and resulting in disabling symptoms. Effective treatments to improve nerve signal conduction are currently limited.

Potassium channel blockers are a group of compounds known to enhance the conduction of nerve impulses, making them a key area of interest in the symptomatic management of conditions such as spinal cord injury, MS, and Alzheimer's disease. Among these, aminopyridines, a subclass of potassium channel blockers, have shown particular promise in treating neurological disorders. One example, 4-aminopyridine (4-AP), also called fampridine, works by inhibiting potassium flow during nerve impulse transmission, effectively aiding in restoring conduction in damaged or demyelinated nerves.

Fampridine is a potassium channel blocker indicated for symptomatic improvement of walking in adults with multiple sclerosis, including relapsing remitting, secondary progressive, progressive relapsing and primary progressive. The chemical name of fampridine is 1,4-dihydropyridin-4-imine and its chemical structure is shown in Formula I.

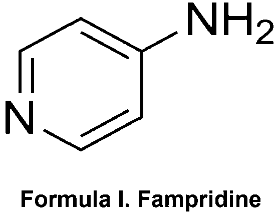

Fampridine is available under the trade name Ampyra from Acorda Therapeutics is available as 10-mg film-coated, sustained-release, white to off-white, oval tablets.

Fampridine has been formulated in sustained-release matrix tablets at various concentrations ranging from 5 to 40 mg.

There is no known cure for MS until now. Current treatments typically focus on accelerating patient's recovery from attacks, slowing the progression of the disease and restraining MS symptoms.

Research in patients with multiple sclerosis (MS), including phase 1, 2 and 3 clinical trials, has shown that the drug fampridine can improve many types of nerve function damaged by the disease, with particular attention paid to its effects on improving walking and leg strength.

WO2017058869A1 discloses sustained release fampridine tablets, which include a core and a coating. The core comprises fampridine, a polyethylene oxide with a molecular weight between about 1,000,000 and 10,000,000, and a mixture comprising polyvinyl acetate and polyvinyl pyrrolidone; and a coating comprises ethylcellulose surrounding said compressed core.

US20170071923A1 provides a sustained release oral dosage form containing fampridine that can be administered once daily. The dosage form includes fampridine as the active pharmaceutical ingredient and the excipients comprising osmotic agents in a tablet core.

EP1732548B2 discloses tablet formulations comprising aminopyridine as an active ingredient. Especially, it discloses used microcrystalline cellulose, magnesium stearate, colloidal silicon dioxide, hydroxypropyl methylcellulose K100LV. In this patent, particularly, preferred diluent is microcrystalline cellulose sold under name Avicel PH 101. There is no information about other types of microcrystalline cellulose and their technical effects in this prior art document.

There are many types of microcrystalline cellulose products on the market, such as PH 101 (average particle size of 40-60 µm, loose density of 0.26-0.32 g/mL), PH 102 (average particle size of 70-100 µm, loose density of 0.28-0.33 g/mL), PH 103 (average particle size of 45-75 µm, loose density of 0.26-0.34 g/mL) and PH 112 (average particle size of 90-140 µm, loose density of 0.28-0.37 g/mL). There are also special types of microcrystalline cellulose products with a higher density or a small particle size, such as PH 301 (the average particle size is 40-60 µm, the loose density is 0.34-0.45 g/ml), PH 302 (the average particle size is 90-140 µm, the loose density is 0.35-0.50 g/ml) and PH 105 (the average particle size is 20-40 µm, the loose density is 0.2-0.3 g/mL).

Prior art includes several documents that describe fampridine in oral pharmaceutical dosage forms. However, these compositions are characterized by their poor flowability and high sensitivity of APIs to moisture caused by excipients in the compositions. Notably, no existing solutions or recommendations effectively address both flowability and moisture resistance simultaneously.

### Detailed description of the Invention

The main object of the present invention is to improve flowability properties of sustained-release composition comprising fampridine. Improved flowability properties of formulation provide high-speed tableting, reducing flow-related issues such as weight variation or inconsistent die filling.

Another object of the present invention is to develop a sustained-release composition comprising fampridine, specifically designed for moisture-sensitive APIs like fampridine, where maintaining low moisture levels is essential to prevent hydrolysis and degradation.

Another object of the present invention is to provide a process for a sustained-release composition comprising fampridine. The process is a simple, rapid, cost effective, timesaving and industrially convenient method.

The invention relates to a sustained-release pharmaceutical composition comprises fampridine homogeneously dispersed in a rate-controlling polymer matrix and at least one diluents, wherein the diluent is microcrystalline cellulose and its average particle size d(0.5) is 70-140 µm and loose density is 0.35-0.46 g/ml.

In the present invention, the diluent used is microcrystalline cellulose, identified by the code name PH-302 with an average particle size d(0.5) ranging from 70 to 140 µm and a loose density of 0.35 to 0.46 g/ml.

Compared to PH-101 microcrystalline cellulose, PH-302 microcrystalline cellulose exhibits higher average particle size and bulk density. The use of PH-302 microcrystalline cellulose as a diluent in the composition enhances the flow properties of the sustained-release formulation containing fampridine and prevents its hydrolysis or degradation caused by exposure to high-moisture-content excipients. Enhancing flow properties facilitates high-speed tableting and minimizes flow-related issues, such as weight variation or inconsistent die filling.

According to another embodiment of the present invention, the amount of microcrystalline cellulose is between 25.0% to 60.0% by weight of the total composition. Preferably, it is between 30.0% to 45.0% by weight of the total composition.

The matrix plays a crucial role in controlling the release rate of the pharmaceutical compound, fampridine. Specifically, the matrix is made of a rate-controlling polymer.

Targeting of drugs to the colon following oral administration has also been accomplished by using rate-controlling polymers ; also they act as binders that swell when hydrated by gastric media and delay absorption. In this invention, we have used cellulose derived polymers as rate-controlling polymers.

Cellulose and its derivatives have demonstrated to be versatile materials with unique chemical structure which provides a good platform for the construction of hydrogel networks with distinctive properties as respects of swelling ability and sensibility to external stimuli. The cellulosic rate-controlling polymer provides the desired dispersion of fampridine throughout the matrix and it imparts chemical and physical stability to the composition while providing prolonged-release profile. This improved dosage stability is particularly important in compositions of the present invention containing low amounts of fampridine. By utilising a cellulosic rate-controlling polymer, stability is achieved while maintaining the desired prolonged-release profile.

Suitable rate-controlling polymer matrix comprises polymers are selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

According to another embodiment of the present invention, the rate-controlling polymer matrix comprises hydroxypropyl methyl cellulose. This excipient provides stability while ensuring the desired sustained-release profile. Especially, a low-viscosity type of HPMC is used, for example; HPMC K100LV.

According to another embodiment of the present invention, the amount of hydroxypropyl methyl cellulose is between 25.0% to 70.0% by weight of the total composition. Preferably, it is between 42.0% to 65.0% by weight of the total composition. More preferably, it is between 52.0% to 65.0% by weight of the total composition.

Since low amounts of fampridine are used in the formulation, it is important to ensure content uniformity. When used in the particle sizes specified below, fampridine helped provide the desired dissolution profile by ensuring content uniformity.

As used here in, 'particle size' means the cumulative volume size distribution as tested by any conventionally accepted method such as the laser diffraction method (i.e. Malvern analysis). The term d(0.9) means the size at which %90 by volume of the particles are finer, the term d(0.5) means the size at which %50 by volume of the particles are finer, the term d(0.1) means the size at which %10 by volume of the particles are finer.

According to another embodiment of the present invention, a sustained-release tablet comprises fampridine and at least one pharmaceutically acceptable excipient wherein a d(0.9) particle size of fampridine is smaller than 120 µm and at least one cellulosic rate-controlling polymer.

According to one embodiment, fampridine has a d(0.9) particle size between 120 µm and 20 µm or between 100 µm and 25 µm or between 85 µm and 30 µm or between 80 µm and 35 µm. Use of particle size in this range ensures content uniformity.

According to another embodiment of the present invention, the amount of fampridine is between 0.1% to 7.0% by weight of the total composition. Preferably, it is between 1.2% to 3.5% by weight of the total composition.

In a preferred embodiment according to the present invention, the sustained-release composition further comprises at least one pharmaceutically acceptable excipient which is selected from lubricants, glidants or mixtures thereof.

Suitable lubricants are selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, , sodium stearyl fumarate, sodium lauryl sulphate or mixtures thereof.

According to another embodiment of the present invention, the lubricant is magnesium stearate.

According to another embodiment of the present invention, the amount of magnesium stearate is between 0.05 to 3.0 by weight of the total composition.

Suitable glidants are selected from the group comprising anhydrous colloidal silicon dioxide, corn starch, talc or mixtures thereof.

According to another embodiment of the present invention, the glidant is anhydrous colloidal silicon dioxide.

According to another embodiment of the present invention, the amount of anhydrous colloidal silicon dioxide is between 0.05 to 3.0 by weight of the total composition. Preferably, it is between 0.1% to 1.5% by weight of the total composition.

According to another embodiment of the present invention, the sustained-release composition comprises;
- Fampridine
- HPMC K100LV
- Microcrystalline cellulose PH302
- Anhydrous colloidal silicon
- Magnesium stearate

The pharmaceutical dosage form comprising the sustained-release composition is a solid pharmaceutical dosage form for oral administration, preferably a tablet, a capsule or a film-coated tablet.

According to another embodiment of the present invention, the sustained-release composition is film coated tablet form. The composition is further comprises a film coating which comprises at least one coating agent.

Suitable coating agents are selected from the group comprising hydroxypropyl methyl cellulose, polymethacrylates, polyalkylacrylates copolymers, hydroxyl propyl methyl cellulose, lactose monohydrate, hydroxypropyl cellulose, polyvinyl alcohol, polyethylene glycol, talc, polyvinyl alcohol-polyethylene glycol copolymers (Kollicoat^{®} IR), ethylcellulose dispersions (Surelease^{®}), polyvinylpyrrolidone, polyvinylpyrrolidone-vinyl acetate copolymer (PVP-VA), all kinds of Opadry^{®}, pigments, dyes, titanium dioxide, iron oxide or mixtures thereof.

According to another embodiment of the present invention, the coating agent is hydroxypropyl methyl cellulose, titanium dioxide, polyethylene glycol or mixtures thereof.

According to one embodiment, the sustained-release tablet has a hardness of between 180 N and 300 N. This provides high chemical and mechanical stability, thus it is not brittle easily Hardness test is done with Erweka Tablet Hardness Tester.

The sustained-release tablet can be prepared using direct compression, wet or dry granulation, hot melt granulation, hot melt extrusion, fluidized bed granulation, extrusion, spheronization, slugging, spray drying or solvent evaporation. In the invention, the sustained-release tablet is obtained by wet granulation or direct compression.

### Example 1:

| **Ingredients** | **Unit Amount (mg)** | **Amount (%)** | **Amount (%)** |
|---|---|---|---|
| Fampridine | 10 | 0.1 - 7.0 | 2.5 |
| HPMC K100LV | 240 | 25.0 - 70.0 | 60.0 |
| Microcrystalline cellulose PH302 | 147.6 | 25.0 - 60.0 | 36.9 |
| Anhydrous colloidal silicon dioxide | 1.60 | 0.05 - 3.0 | 0.4 |
| Magnesium stearate | 0.80 | 0.05 - 3.0 | 0.2 |
| **Total Core Tablet Weight** | **400** | **100.00** | **100.00** |
| OPADRY WHITE Y-1-7000 | 10.00 | | |
| *Hydroxypropyl methyl cellulose 5 cP (Methocel E5 LV) %62,5* | | | |
| *Titanium dioxide%31,25* | | | |
| *Polyethylene glycol 400 %6,25* | | | |
| **Total Coating Tablet Weight** | **410** | | |

A process for example 1;
a) Weighing the active substances and all excipients,
b) Mixing Fampridine and Anhydrous colloidal silicon dioxide homogenously,
c) Adding Microcrystalline cellulose PH302 into the mixture and mixing homogenously,
d) Sieving the mixture through a 0.6 mm round sieve,
e) Granulation with alcohol or water or alcohol/water mixture (70%) and followed by drying the granules at 50°C and sieving them through a 0.6 mm sieve.
f) Adding hydroxypropyl methylcellulose K100LV as the external phase and mixing for 5 min.
g) Sieving Magnesium stearate and adding into the mixture and mixing for 3 min.
h) Pressing the prepared mixture into tablets to ensure the tablet weight meets the specified requirements.
i) Coating the tablets until the desired film-coated tablet weight is achieved.

Another procedure for example 1;
a) Weighing the active substances and all excipients,
b) Mixing of fampridine and anhydrous colloidal silicon dioxide homogeneously,
c) Mixing half of the hydroxypropyl methylcellulose K100LV geometrically into the mixture for 5 minutes,
d) Sieving the mixture through a 0.6-0.8 mm sieve,
e) Adding the remaining half of the hydroxypropyl methylcellulose K100LV to the mixture and stirring for 5 minutes,
f) Adding microcrystalline cellulose PH302, mixing for 10 minutes and sieving.
g) Sieving magnesium stearate, adding to the mixture and mixing for 3 minutes.
h) Pressing the prepared mixture into tablets to ensure that the tablet weight meets the specified requirements.
i) Coating the tablets until the desired film-coated tablet weight is achieved.

## Claims

1. A sustained-release pharmaceutical composition comprising fampridine, rate-controlling polymer matrix and at least one diluents, wherein diluent is microcrystalline cellulose and its average particle size d(0.5) is 70-140 µm and loose density is 0.35-0.46 g/ml.

2. The composition according to claim 1, wherein the amount of microcrystalline cellulose is between 25.0% to 60.0% by weight, preferably between 30.0% to 45.0% by weight of the total composition.

3. The composition according to claim 1, wherein rate-controlling polymer matrix comprising at least one cellulose derived polymer which is selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

4. The composition according to claim 1 or 3, wherein the rate-controlling polymer matrix comprises hydroxypropyl methyl cellulose.

5. The composition according to claim 4, wherein the amount of hydroxypropyl methyl cellulose is between 25.0% to 70.0% by weight of the total composition.

6. The composition according to any preceding claims, wherein fampridine a d(0.9) particle size of fampridine is smaller than 120 µm and at least one cellulosic rate-controlling polymer.

7. The composition according to any preceding claims, wherein fampridine has a d(0.9) particle size between 120 µm and 20 µm or between 100 µm and 25 µm or between 85 µm and 30 µm or between 80 µm and 35 µm.

8. The composition according to any preceding claims, wherein the amount of fampridine is between 0.1% to 7.0% by weight of the total composition.

9. The composition according to any preceding claims, wherein further comprising at least one pharmaceutically acceptable excipient which is selected from lubricants, glidants or mixtures thereof.

10. The composition according to claim 9, wherein lubricants are selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, sodium stearyl fumarate, sodium lauryl sulphate or mixtures thereof.

11. The composition according to claim 9, wherein glidants are selected from the group comprising anhydrous colloidal silicon dioxide, corn starch, talc or mixtures thereof.

12. The composition according to claim 9, wherein comprising;
- Fampridine
- HPMC K100LV
- Microcrystalline cellulose PH302
- Anhydrous colloidal silicon
- Magnesium stearate
